# EUROPEAN PATENT SPECIFICATION

(11) **EP 1 999 487 B1**
(45) Date of publication and mention of the grant of the patent: **16.05.2012**
(21) Application number: 07757887.0
(22) Date of filing: 05.03.2007
(51) Int. Cl.: G01T 1/29, G01T 1/164

(54) **COMPUTED TOMOGRAPHY DATA ACQUISITION APPARATUS AND METHOD**
VORRICHTUNG UND VERFAHREN ZUR ERFASSUNG VON CT-DATEN
PROCEDE ET APPAREIL D'ACQUISITION DE DONNEES DE TOMODENSITOMETRIE

(30) Priority: 16.03.2006 US 767300 P
(43) Date of publication of application: 10.12.2008
(73) Proprietor: Koninklijke Philips Electronics N.V., 5621 BA Eindhoven (NL)
(72) Inventor: ZIEGLER, Andy, 22159 Hamburg (DE); NIELSEN, Tim, 22145 Hamburg (DE)
(74) Representative: Schouten, Marcus Maria
(86) International application number: PCT/US2007/063279
(87) International publication number: WO 2007/109408

(56) References cited:
- EP-A1- 0 948 930
- WO-A-94/24939
- WO-A-2006/064404
- DE-A1- 10 354 214
- US-A1- 2004 081 273

## Description

The present application is directed to the radiographic imaging, and particularly to techniques for reducing the effects of truncation artifacts resulting from detectors having a relatively limited field of view. It finds particular application to x-ray computed tomography (CT), and especially in situations where it is desirable to produce high resolution images of a limited region of interest (ROI).

CT scanners have proven to be invaluable in providing information indicative of the internal structure of an object. In medical imaging, for example, CT scanners are widely used to provide images and other information about the physiology of human patients. Typically, the information generated by a CT scan is reconstructed to generate volumetric data which is in turn presented by way of one or more human readable images.

Recent trends have seen the rapid adoption of multi-slice CT as well as a move to systems having an ever faster rotation speeds. As a result, CT scanners have made increasing inroads in cardiac applications, which typically benefit from improved spatial and temporal resolutions.

Commercially available CT systems traditionally include a generally arcuate radiation sensitive detector. To avoid truncation artifacts in the reconstructed volumetric data, the detector should have a transaxial field of view which is larger than the transaxial dimensions of the objects to be imaged. While these detectors have proven useful in a wide variety of applications, technical and economic considerations typically limit the available spatial resolution.

Flat panel detectors have also been detector. Such detectors typically have a relatively higher resolution than traditional CT detectors. However, technical and economic considerations typically limit the physical size of the detector and thus the available field of view. As a result, flat panel detectors are ordinarily more suitable for use in imaging relatively small objects. While such detectors can be used to image relatively larger objects, the resultant truncation artifacts have offset the benefits provided by the increased spatial resolution. This is especially true in cardiac imaging and other applications which require a relatively high spatial resolution over a relatively small field of view.

EP 0 948 930 A1 is related to acquiring volumetric image data. A gantry includes a large diameter bearing having an outer race and an inner race which surrounds an examination region. An x-ray tube and collimator are mounted to the inner race, as is a flat panel detector and a mechanical mechanism for moving the flat panel detector closer to and further from the examination region. A timing and control circuit controls a motor which indexes the inner race around the examination region, an x-ray power supply which pulses the x-ray tube in a fluoroscopic mode at discrete positions around the examination region, and a read-out circuit which reads out a frame of data after each pulse of the x-ray tube. The read out frames of data are stored in a frame memory and reconstructed by a reconstruction processor into a volumetric image representation for storage in a volume image memory. A video processor reformats individual frames from frame memory or selected portions of volume image representation from the volume image memory into appropriate format for display on a video monitor. A magnification control coordinates adjustment of the collimator and movement of the flat plate detector toward and away from the examination region.

According to US 2004/0081273 A1 cone beam volume CT breast imaging is performed with a gantry frame on which a cone-beam radiation source and a digital area detector are mounted. The patient rests on an ergonomically designed table with a hole or two holes to allow one breast or two breasts to extend through the table such that the gantry frame surrounds that breast. The breast hole is surrounded by a bowl so that the entire breast can be exposed to the cone beam. Spectral and compensation filters are used to improve the characteristics of the beam. A materials library is used to provide x-ray linear attenuation coefficients for various breast tissues and lesions.

In DE 103 54 214 A1 a method and CT scanner are proposed for the production of tomographic section images, in particular X-ray CT images, of a periodically moving object with periodically changing movement and rest phases. For scanning, a number of focus detector combinations with flat detectors are moved on coaxial spiral paths and movement signals from the moving object are measured at the same time in order to detect movement and rest phases. Further, the time correlation between the movement data and the detector output data stored and axial segment image stacks are then reconstructed independently of one another from sub-segments of the spiral paths using the detector output data from each detector which represent a rest phase of the moving object.; Additionally, segment image stacks from the n spiral paths of the n focus detector combinations at the correct time are added up in a complementary angle form and in layers to form 180 DEG tomography section images. The axial segment image stacks are reconstructed in a first step from double-inclined reconstruction planes. Further, in a second step, they are reformatted to produce axial segment image stacks, and detector data from a number of successive movement periods are used for this purpose.

### SUMMARY

Aspects of the present invention address these matters, and others.

According to one aspect of the present invention, a tomographic apparatus is provided as defmed in claim 1.

According to another aspect, a tomography method is provided as defined in claim 7.

According to another aspect of the invention, a computer readable storage medium is provided as defined in claim 11.

Those skilled in the art will appreciate still other aspects of the present invention upon reading and understanding the attached figures and description.

### FIGURES

The present invention is illustrated by way of example and not limitation in the figures of the accompanying drawings, in which like references indicate similar elements and in which:
Figure 1 depicts a CT scanner.
Figure 2 depicts the acquisition geometry of a CT scanner.
Figure 3 is a functional block diagram of a data combiner.
Figures 4a, 4b, 4c, 4d depict projection data.
Figure 5 depicts a technique for using scan data to generating a human readable image.
Figure 6 depicts a technique for using scan data to generate a human readable image having an improved temporal resolution.
Figure 7 depicts a technique for interactively selecting a region of interest.

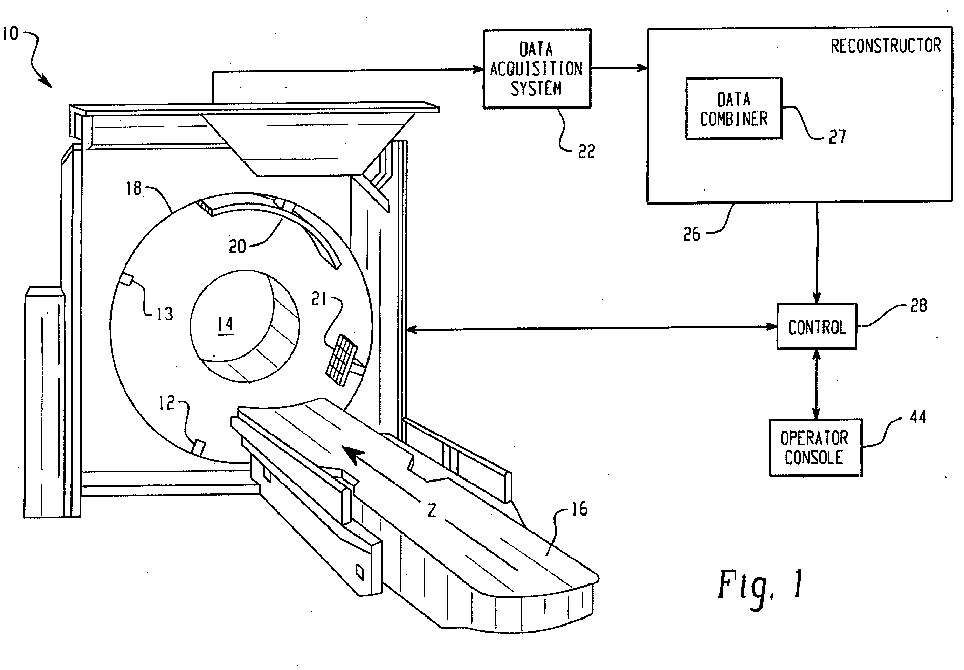

With reference to Figure 1, a CT scanner 10 includes a rotating gantry 18 which rotates about an examination region 14. The gantry 18 supports a first radiation source 12 such as an x-ray tube and a first x-ray sensitive detector 20 which subtends an arc on the opposite side of the examination region 14. The gantry 18 also supports a second x-ray source 13 and a second x-ray sensitive detector 21. X-rays produced by the x-ray sources 12, 13 traverse the examination region 14 and are detected by the detectors 20, 21. The detectors 20, 21 in turn generate respective first and second projection data indicative of the detected radiation.

The first detector 20 is characterized by a relatively low transaxial resolution and a relatively large transaxial field of view. In one implementation, the detector includes an arcuate array of detector elements 100 arranged in a plurality of longitudinal rows or slices and transverse columns. In one implementation, the detector includes 64 or more slices. Each detector element 100 includes a scintillator in optical communication with a photodiode. The photodiodes are preferably fabricated from arrays of back illuminated photodiodes (BIPs), although other photodiode or photodetector technologies can be used. A so-called fourth generation scanner configuration, in which the detector 20 spans an arc of 360 degrees and remains stationary while the x-ray source 12 rotates, as well as flat panel detectors, may also be implemented. Detector having greater or lesser number of slices may likewise be implemented. Depending on the configuration of the detector 20, the first x-ray source 12 generates a beam of radiation having a generally conical, fan, or other desired shape.

The second detector 21 is characterized by a transaxial spatial resolution which is higher and a transaxial field of view which is smaller than that of the first detector 20. The second detector 21 may be implemented as a flat panel detector arranged as a two-dimensional, n x m array of detector elements, although other implementations are possible. For example, the second detector may be implemented in an arcuate array of detector elements similar to those of the first detector 20, but having a relatively higher resolution and having a desired longitudinal extent. The second x-ray source 13 likewise generates a radiation beam consistent with the configuration of the second detector 21.

A data acquisition system 22 preferably located on the rotating gantry 18 receives the projection data generated by the detectors 20, 21 and provides necessary signal conditioning, analog to digital conversion, multiplexing, and like functionality. While illustrated as a single data acquisition system, separate data acquisition systems may be provided for the first and second detectors 20, 21.

A reconstructor 26 reconstructs the data from the detectors 20, 21 to generate volumetric data indicative of radiation attenuation of the object under examination, for example the interior anatomy of a human patient. As will be described in further detail 26, the reconstructor 26 includes a data combiner 27 which uses the data acquired by the lower resolution, larger field of view first detector 20 and the higher resolution, smaller field of view second detector 21 so as to generate a relatively high quality image of a region of interest (ROI) of the object.

A general purpose computer serves an operator console 44. The console 44 includes a human-readable output device such as a monitor or display and an input device such as a keyboard and mouse. Software resident on the console allows the operator to control the operation of the scanner by establishing desired scan protocols, initiating and terminating scans, viewing and otherwise manipulating the volumetric image data, and otherwise interacting with the scanner.

An object support 16 supports an object such as human patient in the examination region 14. The support 16 preferably includes drives which move the support 16 to facilitate the positioning of region(s) of interest of the object in the detector 20, 21 fields of view. The support is also moved in coordination with the rotation of the gantry 18 so as to provide a helical, circular or other desired scanning trajectory.

A controller 28 coordinates the various scan parameters as necessary to carry out a desired scan protocol, including x-ray source 12, 13 parameters, movement of the patient couch 16, and operation of the data acquisition system 26.

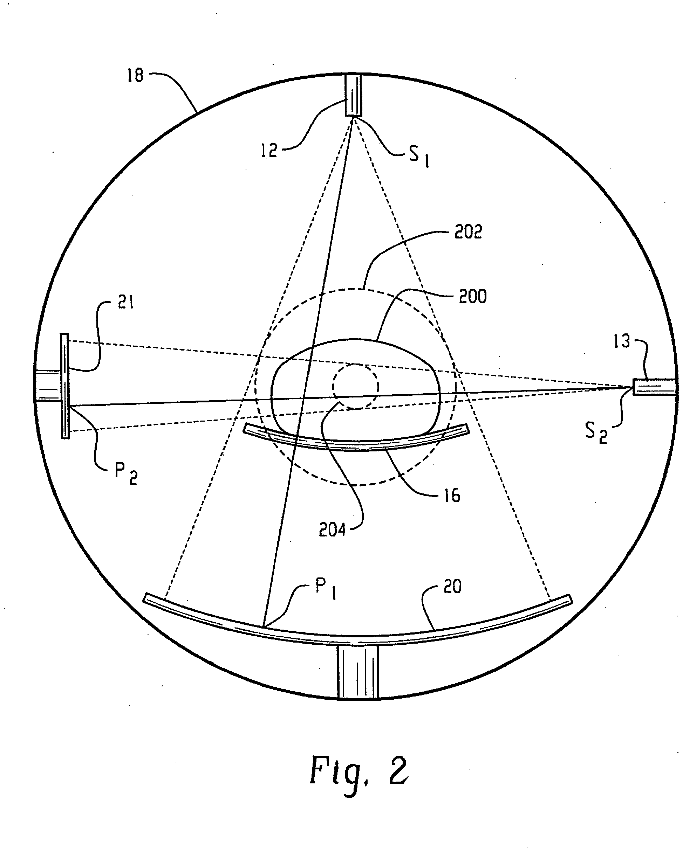

Turning now to Figure 2, the acquisition geometry is shown in greater detail for a given rotating gantry 18 position and object under examination 200. The first detector 20 has a transaxial field of view 202 which is preferably equal to or greater than the maximum transaxial dimension of the object 200. The second detector 21 has a transaxial field of view 204 which is smaller than that of the first detector. Depending on the size of the object 200, the extent of the second detector 21 field of view may also be less than the transaxial extent of the object 200. As will be appreciated by those skilled in the art, volumetric data reconstructed using only the data generated by the second detector would then contain truncation artifacts.

An exemplary projection acquired by the first detector 20 is denoted by the line P₁-S₁, where P₁ represents the position of an exemplary detector element of the first detector 20, and S₁ represents the position of the position of the first x-ray source 12. Similarly, an exemplary projection acquired by the second detector 21 is denoted by the line P₂-S₂, where P₂ represents the position of an exemplary detector element of the second detector 21, and S₂ represents the position of the position of the second x-ray source 13. As the detectors 20, 21 each include a plurality of detector elements, data acquired at each gantry 18 position includes a plurality of projections. Rotation of the gantry 18 and movement of the object support 16 are coordinated so that the detectors 20, 21 traverse a circular, helical, or other desired trajectory about the object 200, thus generating projection data at each of a plurality of positions.

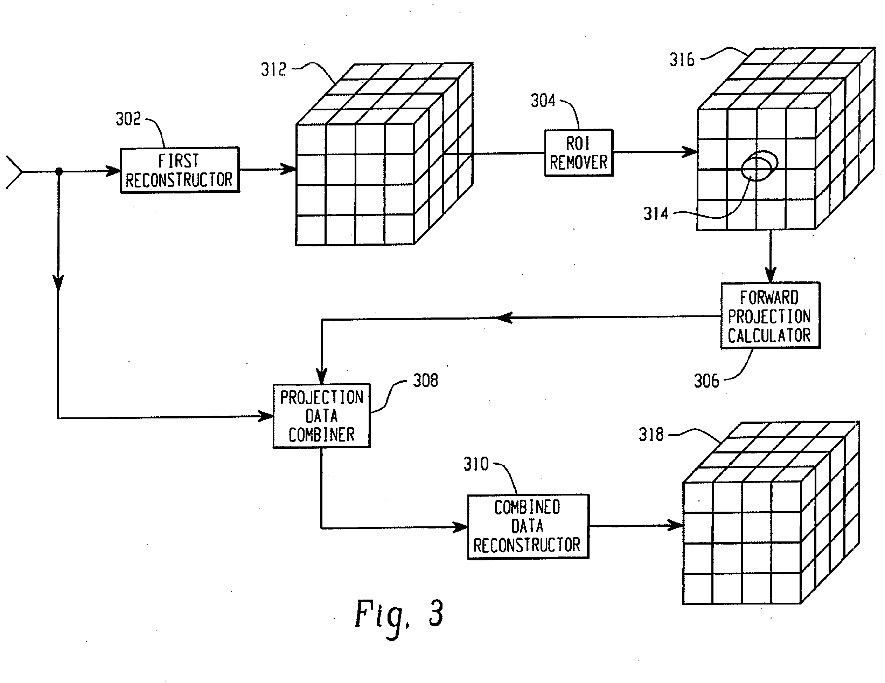

Figure 3 is a functional block diagram of the data combiner 27, which in the illustrated embodiment includes a first reconstructor 302, an ROI filter or remover 304, a forward projection calculator 306, a projection data combiner 308, and a combined data reconstructor 310.

The first reconstructor 302 reconstructs data acquired by the first detector 20 to generate first volumetric data 312 indicative of the object. The reconstruction is typically performed using filtered back projection techniques as are well known to those skilled in the art, although iterative or other suitable reconstruction techniques may also be implemented. As the first volumetric data 312 will subsequently be used to approximate the line integrals of projections outside the field of view of the second detector 21, the first volumetric data 312 may be of lower quality than that generated in a typical diagnostic scan. For example, the reconstruction parameters may be established so that the volumetric data 312 is of a relatively low resolution. The scan parameters may also be selected to produce a relatively low dose, and hence relatively noisier, volumetric data 312. Of course, the scan and reconstruction parameters may be selected so that the volumetric data 312 is of diagnostic quality.

The ROI remover or filter 304 removes an ROI 314 from the volumetric data 312 so as to generate filtered volumetric data 316. In one implementation, the ROI 314 is selected by the user. In such an implementation, the volumetric data 312 may be advantageously displayed on the operator console 44, and the user selects the desired ROI 314 using the mouse and/or keyboard. In another, the ROI 314 determined automatically or semi-automatically using a suitable image processing technique such as segmentation. A particular advantage of such a technique is that the ROI may be selected so as to exclude strong absorption gradients, as may occur when both soft tissue and bone are present in the ROI. The use of relatively fast, approximate cone beam reconstruction techniques, which are typically sensitive to such gradients, is thus facilitated. Moreover, the impact of missing data may also be reduced, particularly in axial reconstructions in which strong absorption gradients can lead to undesirable image artifacts. In still another implementation, the ROI 314 is established to be coextensive with the field of view 204 of the second detector 21. In any case, however, the ROI 314 is preferably located so as to fall within the field of view 204 of the second detector 21.

Voxels falling within the region of interest 314 are filtered or removed from the volumetric data 312, for example by setting them to the value of air (e.g., -1000 HU). To avoid discontinuities in the data, an interpolation or smoothing operation may be performed on voxels near the interface between the ROI 314 and the remaining volumetric data 316.

The forward projection calculator 306 calculates projections through the modified volumetric data 316 corresponding to the second detector 21 trajectory. More particularly, the line integrals through the modified volumetric data 316 are calculated for projections corresponding to those generated by the second detector 21. As the projections do not correspond to the coordinate system of the modified data 316, the projections may be calculated using a high order interpolation technique based on voxels in the neighborhood of the projections.

The projection data combiner 308 combines the data generated by the forward projection calculator 306 with the projection data generated by the second detector 21. More particularly, the various projections generated by the second detector 21 are subtracted from spatially corresponding projections generated by the projection calculator 306.

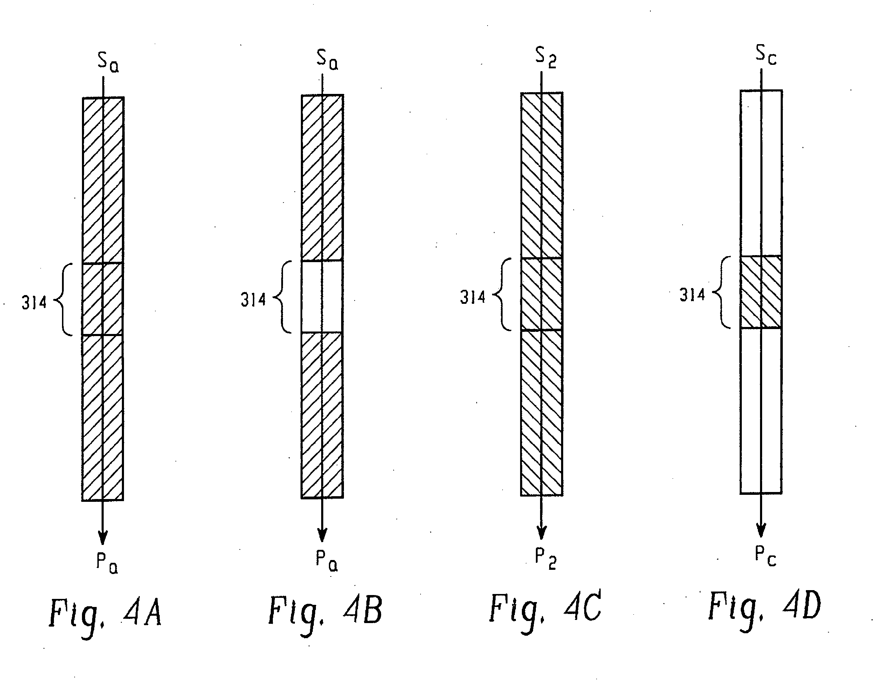

The subtraction process for an exemplary projection is illustrated in Figure 4. Figure 4a depicts an arbitrary projection along path Sₐ-Pₐ through the volumetric data 312. The projection includes attenuation contributions from both inside and outside the region of interest 314 and the field of view 204 of the second detector. Figure 4b depicts the arbitrary projection Sₐ-Pₐ with the contribution from voxels in the region of interest 314 being filtered or removed for processing by the forward projection calculator 306. Figure 4c depicts the projection S₂-P₂ acquired by the second detector 21 along the arbitrary projection Sₐ-Pₐ. The projection, which corresponds to the line integral of the radiation attenuation along the projection S₂-P₂, includes attenuation contributions from both inside and outside the region of interest 314. Figure 4d depicts the projection data as generated by the combiner 308. As can be seen, the attenuation contributions from outside the region of interest are largely cancelled, so that the projection data is indicative primarily of the radiation attenuation in the ROI 314, and hence in the field of view 204 of the second detector 21. Note that the resolution of the first volumetric data, the spatial correspondence between the measured and calculated projections will affect the accuracy of completeness of the cancellation. In any case, truncation artifacts which would ordinarily be generated by the reconstruction of the second detector 21 projection data may advantageously be reduced.

The resultant combined projection data is reconstructed by the combined data reconstructor 310. Again, the reconstruction may be performed using filtered back projection techniques as are well known to those skilled in the art, although iterative or other suitable reconstruction techniques may also be implemented. As the projection data generated by the second detector 21 is typically of relatively high resolution, the reconstruction parameters may be established accordingly. In this regard, it should be noted that the relatively smaller projection matrices resulting from the use of relatively smaller regions of interest can reduce the reconstruction time of an iterative reconstruction, thus improving the attractiveness of iterative techniques.

In one implementation, the various functions described above are implemented via computer readable instructions stored on a disk, memory, or other storage media which are executed by one or more of the computer processors associated with the reconstructor 26. Moreover, some of the functionality, such as that provided by the first data reconstructor and the combined data reconstructor, may be performed using common functions or routines which are executed as desired.

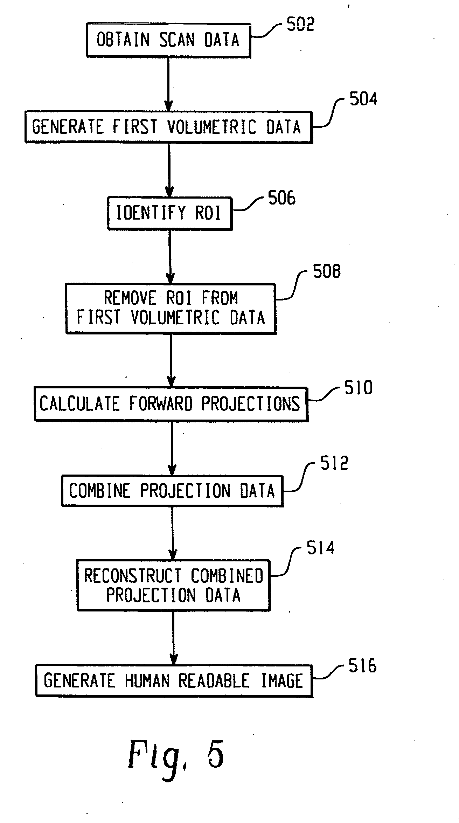

In operation, and with reference to Figure 5, scan data is obtained at step 502, for example by conducting a CT scan of the object using a scanner 10 such as the one illustrated in Figure 1.

At 504, the projection data from the first detector is reconstructed to generate the first volumetric image data 312.

At 506, the ROI is identified.

At 508, the ROI data is removed or filtered from the first volumetric image data so as to generate the modified image data 316.

At 510, the forward projections corresponding to the second detector 21 trajectory are calculated.

At 512, the projection data from the second detector 21 is combined with the calculated projection data.

At 514, the resultant projection data is reconstructed to generate the volumetric image data 318.

At 516, a human readable image indicative of the volumetric image data is generated and displayed, for example on a monitor associated with the operator console 44. As is well known in the art, the human readable image(s) can take various forms, for example including one or more image slices, volume rendered images, or the like.

In this regard, it should be noted that steps 508, 510, and 512 need not be performed in temporal sequence for all projections in the data set. More particularly, the forward projections may be identified and calculated, the ROI removed, and the projection data combined on a projection-by-projection basis, with the process repeated for each desired projection. Moreover, the projections may be performed retrospectively using previously acquired data.

Other variations are also possible. As illustrated in Figure 1, the first 20 and second 21 detectors and the respective x-ray sources 12, 13 are angularly offset by approximately 90°, thus generating additional data as compared to a scanner having only a single detector. The data from the first 20 and second 21 detectors may be combined to generate volumetric data having a relatively higher temporal resolution, for example in the imaging of cyclically moving objects such as the heart.

At step 602, combined projection data is generated as described above in relation to Figures 3 and 5, with the region in the vicinity of the heart selected as the ROI. As will be appreciated, two projection data sets are thus available: the projection data generated by the first detector 20, and the combined projection data.

At step 604, temporally corresponding projections, such as those obtained at a desired cardiac phase, are selected from the respective data sets.

At step 606, the selected projections are reconstructed to generate volumetric data indicative of the desired cardiac phase.

At step 608, the process is repeated as desired for additional cardiac phases.

The desired human readable image(s) are generated and displayed as desired at step 610.

Where the detectors 20, 21 are offset by 90°, the temporal resolution is improved by about a factor of two over that obtained using a scanner having a single detector. Where the second detector 21 has a relatively higher spatial resolution than that of the first detector 20, the reconstructed image also has a higher resolution than would be obtained in a scanner having a second detector which has a spatial resolution similar to that of the first detector 20. Moreover, the improved temporal resolution allows the use of a narrower gating window, thereby reducing blurring in the reconstructed image.

Information from the first 20 and second 21 detectors may also be used in multiple energy or spectral imaging in which spectrally coded projections are generated. Such projections are typically generated by varying the x-ray source 12, 13 voltage from view to view, or through the use of spectral or energy resolving detectors which provide outputs indicative of radiation detected in more than one energy range.

In particular, the spectral information can be used to differentiate between multiple material base functions. In one implementation, both the first 20 and second 21 detectors produce spectrally coded data. In another, only one of the detectors 20, 21 provides spectral information. In any case, the material base functions may be separated in an optimal or otherwise desired fashion.

As one example, a soft tissue region is selected as the ROI. The first detector 20 works with a spectral coding that provides separation between bone and soft tissue base functions. The second detector 21 may be optimized for another contrast, for example, the separation of contrast agent and soft tissue base functions. As the base functions include the energy dependence of the line integrals, the measurement from the first detector 20 can be used to process the second detector 21 measurements. Moreover, the subtraction of bone from the second detector 21 measurements can be used to reduce artifacts which would otherwise appear in the reconstruction, particularly where strong absorption gradients are located near the ROI 314.

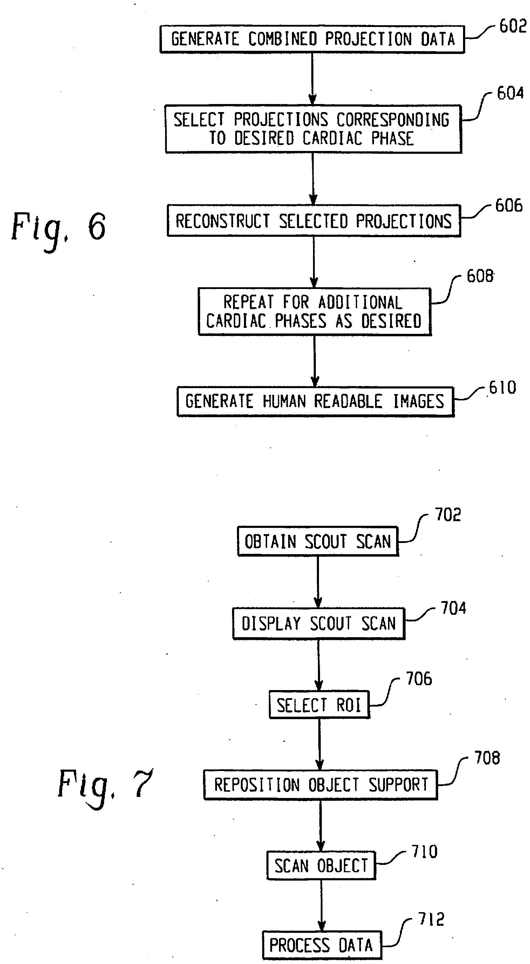

As still another variation, the ROI 314 may be selected interactively. With reference to Figure 7, a scout or other low resolution scan is obtained at step 702. At 704, the scout scan is displayed, for example on the monitor associated with the operator console 44. At 706, the operator identifies the desired ROI 304. The controller 28 uses this information to adjust the position of the object support 16 so that the ROI is located, and ideally centered, in the field of view of the second detector 21. The object is then scanned at 710, and the data is processed as describe above at 712. As the field of view of the second detector 21 is typically centered at the center of rotation of the gantry 18, such a procedure is especially usefully in situations where the ROI is offset from the center of the object under examination or otherwise relatively large in relation to the field of view of the second detector 21.

It should also be noted that the detectors 20, 21 and their respective sources may be offset by angles other than 90°. Moreover, one of the x-ray sources may be omitted, with the second detector 21 being centered in otherwise partially angularly coextensive with the first detector 20. In such an implementation, the data from both the first 20 and second 21 detectors is be used to generate the first volumetric data 312. Further processing of the projection data would then occur as described above.

## Claims

1. A tomographic apparatus comprising:
a first radiation sensitive detector (20) which generates first projection data indicative of an object disposed in an examination region (14);
a second radiation sensitive detector (21) which generates second projection data indicative of the object, wherein the second detector has a second transaxial field of view (204), and wherein the second transaxial view is smaller than a transaxial dimension of the object, whereby volumetric data reconstructed using the second projection data would contain truncation artifacts;
means (304, 306, 308) for correcting the second projection data so as to reduce the truncation artifacts, wherein the correction is a function of the first projection data;
a corrected data reconstructor (310) which generates volumetric data indicative of the corrected second projection data,
wherein the means for correcting include
a first reconstructor (302) which generates first volumetric data (312) indicative of the first projection data;
**characterized in that** the means for correcting further include
an ROI filter (304) which filters the first volumetric data so as to remove an ROI therefrom;
a projection calculator (306) which calculates a plurality of projections through the filtered first volumetric data; and
a projection combiner (308) which combines the second projection data and the calculated projections for correcting the second projection data.

2. The apparatus of claim 1 wherein the first detector has a first transaxial field of view (202) and a first resolution, wherein the second detector has a second resolution, wherein the second transaxial field of view is smaller than the first transaxial field of view, and wherein the second resolution is greater than the first resolution.

3. The apparatus of claim 2 wherein the object includes a beating heart, the apparatus includes means for selecting projections from the first projection data and the corrected second projection data as a function of the cardiac phase, and the corrected data reconstructor generates volumetric data indicative of the selected projections.

4. The apparatus of claim 2 wherein the first detector includes an arcuate x-ray detector and wherein the second detector includes a flat panel x-ray detector.

5. The apparatus of claim 1 wherein the projection combiner (310) subtracts a calculated projection from a spatially corresponding projection of the second projection data.

6. The apparatus of claim 1 wherein the first and second detectors are x-ray detectors and wherein the apparatus includes a first x-ray source 12 disposed across the examination region from the first detector and a second x-ray source 13 disposed across the examination region form the second detector.

7. A tomography method comprising:
receiving first projection data generated by a first radiation sensitive detector (20), wherein the projection data is indicative of an interior of an object, and wherein the object has a transaxial dimension;
receiving second projection data generated by a second radiation sensitive detector (21), wherein the second projection data is indicative of the interior of the object, wherein the second detector has a second transaxial field of view (204), and wherein the second transaxial view is smaller than a transaxial dimension of the object, whereby volumetric data reconstructed using the second projection data would contain truncation artifacts;
correcting the second projection data so as to reduce the truncation artifacts, wherein the correction is a function of the first projection data;
reconstructing the corrected second projection data;
generating a human readable image indicative of the reconstructed data,
the step of correcting the second projection data wherein includes:
reconstructing the first projection data to generate first volumetric data (312), **characterized in that** the step of correcting the second projection data further includes filtering the first volumetric data so as to remove an ROI therefrom, calculating a plurality of first projections through the filtered first volumetric data, and
combining the second projection data and the calculated projections for correcting the second data.

8. The method of claim 7 including identifying a region of interest (314) in the first volumetric data and wherein the first projections do not include a contribution from the region interest.

9. The method of claim 8 wherein the second projection data includes a plurality of second projections and including:
subtracting a calculated projection from a second projection;
repeating the step of subtracting for each of a plurality of second projections.

10. The method of claim 7 wherein the first projection data includes a plurality of projections and wherein reconstructing includes:
selecting temporally corresponding projections in the first projection data and the corrected second projection data; and
reconstructing the selected projections.

11. A computer readable storage medium containing instructions which, when executed by a computer, cause the computer to carry out the steps of the method of claim 7.

## Patentansprüche

1. Tomographiegerät mit:
einem ersten strahlungsempfindlichen Detektor (20), der erste Projektionsdaten erzeugt, die ein Objekt zeigen, das in einer Untersuchungsregion (14) angeordnet ist,
einem zweiten strahlungsempfindlichen Detektor (21), der zweite Projektionsdaten erzeugt, die das Objekt zeigen, wobei der zweite Detektor über ein zweites transaxiales Messfeld (204) verfügt und wobei das zweite transaxiale Messfeld kleiner als eine transaxiale Dimension des Objekts ist, wobei anhand der zweiten Projektionsdaten rekonstruierte volumetrische Daten Truncation-Artefakte enthalten würden,
Mitteln (304, 306, 308) zum Korrigieren der zweiten Projektionsdaten zur Reduzierung der Truncation-Artefakte, wobei die Korrektur von den ersten Projektionsdaten abhängt,
einer Rekonstruktionseinheit (310) für korrigierte Daten, die volumetrische Daten erzeugt, die die korrigierten zweiten Projektionsdaten zeigen,
wobei die Mittel zum Korrigieren Folgendes umfassen:
eine erste Rekonstruktionseinheit (302), die erste volumetrische Daten (312) erzeugt, die die ersten Projektionsdaten zeigen,
**dadurch gekennzeichnet, dass** die Mittel zum Korrigieren ferner Folgendes umfassen:
ein ROI-Filter (304), das die ersten volumetrischen Daten derart filtert, dass eine interessierende Region (ROI) daraus entfernt wird,
eine Projektionsberechnungseinheit (306), die eine Vielzahl von Projektionen durch die gefilterten ersten volumetrischen Daten berechnet, und
eine Projektionskombinationseinheit (308), die die zweiten Projektionsdaten und die berechneten Projektionen kombiniert, um die zweiten Projektionsdaten zu korrigieren.

2. Gerät nach Anspruch 1, wobei der erste Detektor ein erstes transaxiales Messfeld (202) und eine erste Auflösung aufweist, wobei der zweite Detektor eine zweite Auflösung aufweist, wobei das zweite transaxiale Messfeld kleiner als das erste transaxiale Messfeld ist und wobei die zweite Auflösung höher als die erste Auflösung ist.

3. Gerät nach Anspruch 2, wobei das Objekt ein schlagendes Herz enthält, wobei das Gerät Mittel zum Auswählen von Projektionen aus den ersten Projektionsdaten und den korrigierten zweiten Projektionsdaten in Abhängigkeit von der Herzphase umfasst und die Rekonstruktionseinheit für korrigierte Daten volumetrische Daten erzeugt, die die ausgewählten Projektionen zeigen.

4. Gerät nach Anspruch 2, wobei der erste Detektor einen bogenförmigen Röntgendetektor umfasst und wobei der zweite Detektor einen Flachröntgendetektor umfasst.

5. Gerät nach Anspruch 1, wobei die Projektionskombinationseinheit (310) eine berechnete Projektion von einer räumlich entsprechenden Projektion der zweiten Projektionsdaten subtrahiert.

6. Gerät nach Anspruch 1, wobei der erste und der zweite Detektor Röntgendetektoren sind und wobei das Gerät eine erste Röntgenquelle 12 umfasst, die in der Untersuchungsregion dem ersten Detektor gegenüberliegend angeordnet ist, und eine zweite Röntgenquelle 13 umfasst, die in der Untersuchungsregion dem zweiten Detektor gegenüberliegend angeordnet ist.

7. Tomographieverfahren, das Folgendes umfasst:
Empfangen von ersten Proj ektionsdaten, die von einem ersten strahlungsempfindlichen Detektor (20) erzeugt werden, wobei die Projektionsdaten ein Inneres eines Objekts zeigen und wobei das Objekt eine transaxiale Dimension aufweist,
Empfangen von zweiten Projektionsdaten, die von einem zweiten strahlungsempfindlichen Detektor (21) erzeugt werden, wobei die zweiten Projektionsdaten das Innere des Objekts zeigen, wobei der zweite Detektor über ein zweites transaxiales Messfeld (204) verfügt und wobei das zweite transaxiale Messfeld kleiner als eine transaxiale Dimension des Objekts ist, wobei anhand der zweiten Projektionsdaten rekonstruierte volumetrische Daten Truncation-Artefakte enthalten würden,
Korrigieren der zweiten Projektionsdaten zur Reduzierung der Truncation-Artefakte, wobei die Korrektur von den ersten Projektionsdaten abhängt,
Rekonstruieren der korrigierten zweiten Projektionsdaten,
Erzeugen eines menschenlesbaren Bildes, das die rekonstruierten Daten zeigt,
wobei der Schritt des Korrigierens der zweiten Projektionsdaten Folgendes umfasst:
Rekonstruieren der ersten Projektionsdaten zum Erzeugen von ersten volumetrischen Daten (312),
**dadurch gekennzeichnet, dass** der Schritt des Korrigierens der zweiten Projektionsdaten ferner Folgendes umfasst:
Filtern der ersten volumetrischen Daten derart, dass eine interessierende Region (ROI) daraus entfernt wird,
Berechnen einer Vielzahl von ersten Projektionen durch die gefilterten ersten volumetrischen Daten und
Kombinieren der zweiten Projektionsdaten und der berechneten Projektionen, um die zweiten Daten zu korrigieren.

8. Verfahren nach Anspruch 7, das das Identifizieren einer interessierenden Region (314) in den ersten volumetrischen Daten umfasst und wobei die ersten Projektionen keinen Beitrag aus der interessierenden Region enthalten.

9. Verfahren nach Anspruch 8, wobei die zweiten Projektionsdaten eine Vielzahl von zweiten Projektionen enthalten und das Verfahren Folgendes umfasst:
Subtrahieren einer berechneten Projektion von einer zweiten Projektion,
Wiederholen des Subtrahierschrittes für jede einer Vielzahl von zweiten Projektionen.

10. Verfahren nach Anspruch 7, wobei die ersten Projektionsdaten eine Vielzahl von Projektionen umfassen und wobei das Rekonstruieren Folgendes umfasst:
Auswählen von zeitlich entsprechenden Projektion in den ersten Projektionsdaten und den korrigierten zweiten Projektionsdaten und
Rekonstruieren der ausgewählten Projektionen.

11. Computerlesbares Speichermedium, das Befehle enthält, die bei Ausführung durch einen Rechner den Rechner veranlassen, die Schritte des Verfahrens nach Anspruch 7 auszuführen.

## Revendications

1. Appareil de tomographie comprenant :
un premier détecteur sensible au rayonnement (20) qui génère des premières données de projection indicatives d'un objet placé dans une zone d'examen (14) ;
un second détecteur sensible au rayonnement (21) qui génère des secondes données indicatives de l'objet, dans lequel le second détecteur a un second champ de vision transversal (204), et dans lequel la seconde vision transversale est plus petite que la dimension transversale de l'objet, de manière à ce que les données volumétriques reconstruites en utilisant les secondes données de projection contiendraient des artéfacts de troncature ;
des moyens (304, 306, 308) de correction des secondes données de projection afin de réduire les artéfacts de troncature, dans lesquels la correction est une fonction des premières données de projection ;
un reconstructeur de données corrigées (310) qui génère des données volumétriques indicatives des secondes données de projection corrigées,
dans lequel les moyens de correction comprennent :
un premier reconstructeur (302) qui génère les premières données volumétriques (312) indicatives des premières données de projection,
**caractérisé en ce que** les moyens de corrections incluent en outre :
un filtre RI (304) qui filtre les premières données volumétriques afin de supprimer une région d'intérêt RI en provenance de celles-ci ;
un calculateur de projection (306) qui calcule une pluralité de projections via les premières données volumétriques filtrées ; et
un combinateur de projection (308) qui combine les secondes données de projection et les projections calculées pour corriger les secondes données de projection.

2. Appareil selon la revendication 1 dans lequel le premier détecteur a un premier champ de vision transversal (202) et une première résolution, dans lequel le second détecteur a une seconde résolution, dans lequel le second champ de vision transversal est plus petit que le premier champ de vision transversal et dans lequel la seconde résolution est supérieure à la première résolution.

3. Appareil selon la revendication 2 dans lequel l'objet inclut un coeur qui bat, l'appareil inclut des moyens pour sélectionner les projections des premières données de projection et des données corrigées de la seconde projection comme fonction de la phase cardiaque, et le reconstructeur de données corrigées génère des données volumétriques indicatives des projections sélectionnées.

4. Appareil selon la revendication 2 dans lequel le premier détecteur inclut un détecteur arqué de rayons X et dans lequel le deuxième détecteur inclut un détecteur plat de rayons X.

5. Appareil selon la revendication 1 dans lequel le combinateur de projection (310) soustrait une projection calculée d'une projection correspondante spatialement des données de la seconde projection.

6. Appareil selon la revendication 1 dans lequel les premier et second détecteurs sont des détecteurs de rayons X et dans lequel l'appareil inclut une première source de rayons X 12 placée dans le champ de la région d'examen soumise au premier détecteur et une deuxième source de rayons X 13 placée dans le champ de la région d'examen soumise au second détecteur.

7. Méthode de tomographie comprenant :
la réception de premières données de projection générées par un premier détecteur sensible au rayonnement (20), dans laquelle les données de projection sont indicatives d'un intérieur d'un objet et dans laquelle l'objet a une dimension transversale ;
la réception de secondes données de projection par un second détecteur sensible au rayonnement (21), dans laquelle les secondes données de projection sont indicatives de l'intérieur de l'objet, dans laquelle le second détecteur a un second champ de vision transversal (204), et dans laquelle la seconde vue transversale est plus petite que la dimension transversale de l'objet, de manière à ce que les données volumétriques reconstruites utilisant les secondes données de projection contiendraient des artéfacts de troncature ;
la correction des secondes données de projection de manière à réduire les artéfacts de troncature, dans laquelle la correction est une fonction des premières données de projection ;
la reconstruction des secondes données de projection corrigées ;
la formation d'une image lisible par l'homme indicative des données reconstruites,
dans laquelle l'étape de correction des secondes données de projection inclut :
la reconstruction des premières données de projection pour générer les premières données volumétriques (312),
**caractérisée en ce que** l'étape de correction des secondes données de projection inclut en outre :
le filtrage des premières données volumétriques de manière à en retirer une région d'intérêt,
le calcul d'une pluralité de premières projections via les premières données volumétriques filtrées, et
la combinaison des secondes données de projection et des projections calculées pour la correction des secondes données.

8. Méthode selon la revendication 7 incluant l'identification d'une région d'intérêt (314) dans les premières données volumétriques et dans laquelle les premières projections n'incluent pas une contribution de la région d'intérêt.

9. Méthode selon la revendication 8 dans laquelle les secondes données de projection incluent une pluralité de secondes projections et inclut :
la soustraction d'une projection calculée à partir d'une seconde projection ;
la répétition de l'étape de soustraction pour chacune d'une pluralité des secondes projections.

10. Méthode selon la revendication 7, dans laquelle les premières données de projection incluent une pluralité de projections et dans laquelle la reconstruction inclut :
la sélection temporelle des projections correspondantes dans les premières données de projection et les secondes données de projection corrigées ; et
la reconstruction des projections sélectionnées.

11. Support de stockage lisible par ordinateur contenant les instructions qui, lorsqu'elles sont exécutées par ordinateur, conduisent l'ordinateur à exécuter les étapes de la revendication 7.
